(19) Europäisches Patentamt
European Patent Office
Office européen des brevets

(11) Veröffentlichungsnummer: **0 090 309**
**A1**

(12) # EUROPÄISCHE PATENTANMELDUNG

(21) Anmeldenummer: **83102768.5**

(22) Anmeldetag: **21.03.83**

(51) Int. Cl.³: **C 07 D 333/38**, A 01 N 47/36

(30) Priorität: **31.03.82 DE 3211851**

(43) Veröffentlichungstag der Anmeldung: **05.10.83 Patentblatt 83/40**

(84) Benannte Vertragsstaaten: **DE FR GB IT**

(71) Anmelder: **BASF Aktiengesellschaft, Carl-Bosch-Strasse 38, D-6700 Ludwigshafen (DE)**

(72) Erfinder: **Acker, Rolf-Dieter, Dr., Tuchbleiche 8, D-6906 Leimen (DE)**
Erfinder: **Rossy, Phillip A., Dr., Van-Leyden-Strasse 17, D-6700 Ludwigshafen (DE)**
Erfinder: **Hamprecht, Gerhard, Dr., Rote-Turm-Strasse 28, D-6940 Weinheim (DE)**
Erfinder: **Wuerzer, Bruno, Dr. Dipl.-Landwirt, Ruedigerstrasse 13, D-6701 Otterstadt (DE)**

(54) **Dihydrothiophen-carbonester, Verfahren zu ihrer Herstellung und ihre Verwendung zur Bekämpfung unerwünschten Pflanzenwuchses.**

(57) Dihydrothiophen-carbonester der Formel

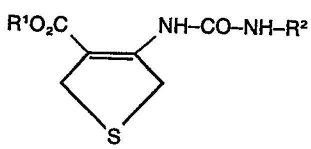

in der

$R^1$ Wasserstoff, Alkyl, Alkenyl, Alkinyl, Halogenalkyl, Alkoxyalkyl, Alkylthioalkyl, Cycloalkyl, gegebenenfalls durch Halogen oder Alkyl substituiertes Phenyl, oder gegebenenfalls durch Halogen substituiertes Benzyl und

$R^2$ Alkyl, Alkenyl, Alkinyl, Halogenalkyl, Alkoxyalkyl, Alkylthioalkyl, Cycloalkyl, gegebenenfalls durch Halogen oder Alkyl substituiertes Phenyl, oder gegebenenfalls durch Halogen substituiertes Benzyl bedeuten.

Verfahren zu ihrer Herstellung und ihre Verwendung zur Bekämpfung unerwünschten Pflanzenwuchses.

EP 0 090 309 A1

Dihydrothiophen-carbonester, Verfahren zu ihrer Herstellung und ihre Verwendung zur Bekämpfung unerwünschten Pflanzenwuchses

Die Erfindung betrifft Dihydrothiophen-carbonester, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Es ist bekannt, daß Thiophenderivate herbizid wirksam sind (US-PS 3 828 001).

Es wurde gefunden, daß Dihydrothiophencarbonester der Formel

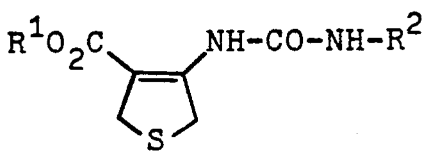

(I),

in der
$R^1$ Wasserstoff, $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl, oder gegebenenfalls durch Halogen substituiertes Benzyl und
$R^2$ $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl, oder gegebenenfalls durch Halogen substituiertes Benzyl bedeuten, herbizid wirksam und für eine Reihe von Kulturpflanzen verträglicher sind als bekannte Thiophencarbonester.

H/ro

$R^1$ und $R^2$ in Formel I bedeuten unverzweigtes oder verzweigtes $C_1$-$C_{10}$-Alkyl, vorzugsweise $C_1$-$C_4$-Alkyl, unverzweigtes oder verzweigtes $C_2$-$C_{10}$-Alkenyl, vorzugsweise $C_3$-$C_4$-Alkenyl, unverzweigtes oder verzweigtes $C_2$-$C_{10}$-Alkinyl, vorzugsweise $C_3$-$C_4$-Alkinyl, unverzweigtes oder verzweigtes $C_1$-$C_{10}$-Halogenalkyl, vorzugsweise $C_1$-$C_4$-Halogenalkyl, unverzweigtes oder verzweigtes $C_2$-$C_{10}$-Alkoxy- oder Alkylthioalkyl, vorzugsweise $C_2$-$C_4$-Alkoxy- oder Alkylthioalkyl, oder $C_3$-$C_7$-Cycloalkyl, beispielsweise Ethyl, n-Propyl, Isopropyl, n-Butyl, Isobutyl, sec-Butyl, tert.-Butyl, n-Pentyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-n-Hexyl, tert.-Amyl, Vinyl, Allyl, Methallyl, Crotyl, 2-Ethyl-hexen-2-yl, Hexen-5-yl, 2-Methyl-buten-2-yl, 2-Methyl-buten-1-yl-3, Butin-1-yl-3, Butin-2-yl, Buten-1-yl-3, Propargyl, 2-Methyl-buten-2-yl-4, 2-Methyl-buten-2-yl-4, 3-Methyl-buten-1-yl-3, 2-Chlorethyl, 2-Chlor-n-propyl, 3-Chlor-n-propyl, 2-Chlorisopropyl, 1-Chlormethyl-n-propyl, 2-Chlorbutyl-3, 2-Chlor-2-methyl-n-propyl, 2-Fluorbutyl-3, 2-Fluor-2-methyl-n-propyl, 2-Fluorisopropyl, Chlor-tert-butyl, 2,2,2-Trifluorethyl, Methoxyethyl, Ethoxyethyl, 3-Methoxy-n-propyl, Methoxyisopropyl, 3-Methoxy-n-butyl, 1-Methoxy-butyl-2, Ethoxy-tert-butyl, Methoxy-tert-butyl, 2-Methoxy-butyl, 4-Methoxy-n-butyl, Methylmercapto-ethyl, Ethylmercapto-ethyl, 3-Methylmercapto-n-propyl, 3-Methylmercapto-n-butyl, 1-Methylmercapto-butyl-2, Methylmercapto-tert-butyl, 2-Methylmercapto-n-butyl, Cyclopropyl, Cyclopentyl, Cyclohexyl, Cycloheptyl.

$R^1$ und $R^2$ können auch einen gegebenenfalls am Phenylring durch Halogen, wie Fluor, Chlor, Brom, Jod, substituierten Benzylrest, wie Benzyl, 2,6-Dichlorbenzyl, 2-Chlor-6-fluorbenzyl, 2,6-Difluorbenzyl, oder einen gegebenenfalls durch Halogen, wie Fluor, Chlor, Brom, Iod, oder $C_1$-$C_4$-

-Alkyl substituierten Phenylrest, wie Phenyl, 4-Chlorphenyl, 2,4-Dichlorphenyl, 4-Isopropylphenyl, 4-tert.-Butylphenyl, bedeuten.

Bevorzugt sind Verbindungen der Formel I, bei denen $R^1$ und $R^2$ $C_1$-$C_4$-Alkyl bedeuten.

Man erhält die Dihydrothiophen-carbonester der Formel I
a) durch Umsetzung von Ketoestern der Formel

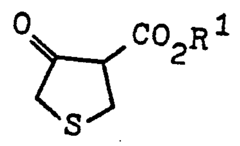

(II),

in der
$R^1$ die obengenannten Bedeutungen hat, mit Harnstoffen der Formel

$$R^2-NH-\overset{O}{\overset{\|}{C}}-NH_2 \qquad (III),$$

in der
$R^2$ die obengenannten Bedeutungen hat,

oder b) durch Umsetzung von Aminoverbindungen der Formel

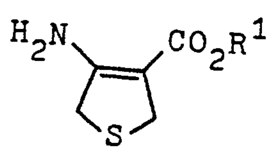

(IV),

in der
$R^1$ die obengenannten Bedeutungen hat, oder ihrer Salze mit einem Isocyanat der Formel

$R^2$-NCO (V),

in der
$R^2$ die obengenannten Bedeutungen hat.

Die Verfahrensvariante a) wird bei einer Temperatur im Bereich zwischen 0 und 150°C, vorzugsweise 50 und 120°C, gegebenenfalls unter Zusatz eines inerten organischen Lösungsmittels durchgeführt. Zweckmäßigerweise wird dem Reaktionsgemisch ein Kondensationsmittel zugesetzt, beispielsweise p-Toluolsulfonsäure, Phosphorsäure, Polyphosphorsäure oder Schwefelsäure. Die Menge an Kondensationsmittel beträgt 0,1 bis 20 Mol.%, bezogen auf Verbindung II.

Zur Erhöhung der Ausbeute kann das entstehende Wasser azeotrop abdestilliert werden. Verbindung II kann in einem Überschuß oder Unterschuß von bis zu 25 Mol.%, bezogen auf Verbindung III, eingesetzt werden.

Ketoester der Formel II, in der $R^1$ Methyl bedeutet, sind bekannt (J. Org. Chem. 45, 617 (1980)). Ketoester der Formel II, in der $R^1$ die für Formel I genannten Bedeutungen, mit Ausnahme von Methyl und Wasserstoff, hat, werden durch Umesterung von $C_1$-$C_3$-Alkylestern der Formel II mit Hydroxylverbindungen der Formel $R^1OH$, in der $R^1$ die für Formel I genannten Bedeutungen, mit Ausnahme von Methyl und Wasserstoff, hat, erhalten.

Bei dieser Reaktion werden zweckmäßigerweise basische oder saure Katalysatoren in Mengen von 0,1 bis 20 Mol.%, bezogen auf Verbindung II, zugesetzt.

Geeignete saure Katalysatoren sind beispielsweise anorganische Säuren, wie Salzsäure, Schwefelsäure, Phosphorsäure, Polyphosphorsäure, oder auch aromatische Carbon-

säuren oder Sulfonsäuren, insbesondere p-Toluolsulfonsäure. Als basische Katalysatoren kommen tertiäre Amine, Erdalkaliverbindungen, Ammoniumverbindungen und Alkaliverbindungen sowie entsprechende Gemische in Betracht. Auch Zinkverbindungen können verwendet werden. Beispiele hierfür sind: Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, beta-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Außer den vorgenannten anorganischen Basen kommen außerdem z.B. Natriumpropionat, Natriumbutyrat, Natriumisobutyrat, Kaliumformiat, Kaliumacetat, Kaliumpropionat, Kalium-

butyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1.2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat in Betracht.

Den Ketoester der Formel II, in der $R^1$ Ethyl bedeutet, erhält man beispielsweise durch folgende Umsetzung:

20 Gew.-Teile 3-Keto-1,5-dihydro-thiophen-4-carbonsäure-methylester, 57,8 Gew.-Teile Ethanol und 1 Gew.-Teil p-Toluolsulfonsäure werden 50 Stunden lang unter Rückfluß gekocht. Bei der anschließenden Destillation erhält man 17,1 Gew.-Teile 3-Keto-1,5-dihydro-thiophen-4-carbonsäure-ethylester vom Fp. 106 bis 109°C/1,3 mbar.

Die Verfahrensvariante b) wird mit ungefähr stöchiometrischen Substanzmengen, d.h. in einem Mengenverhältnis von etwa 0,8 bis 1,2 Mol Verbindung IV zu Verbindung V, gegebenenfalls in Gegenwart eines inerten organischen Lösungsmittels bei einer Temperatur von -20 bis +50°C durchgeführt. Falls Verbindung IV als Salz vorliegt, kann eine Base zugesetzt werden. Es kann dann entweder das freie Amin isoliert werden, oder es werden Verbindungen der Formel V direkt zugegeben. Nach dem Einengen der Lösung reinigt man die Verbindungen der Formel I durch Umkristallisation oder Chromatographie.

Geeignete Basen sind die für die Verfahrensvariante a) aufgeführten basischen Katalysatoren sowie die dort genannten organischen Säuren.

Als Lösungsmittel kommen für beide Verfahren z.B. Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, beta,beta'-Dichlordiethylether; Nitrokohlenwasserstoffe, wie Nitromethan, Nitroethan, Nitrobenzol, o-, m-, p-Chlornitrobenzol, o-Nitrotoluol; Nitrile, wie Acetonitril, Butyronitril, Isobutyronitril, Benzonitril, m-Chlorbenzonitril; aliphatische, cycloaliphatische oder aromatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan, Toluol, o-, m-, p-Xylol, Tetralin; Ester, z.B. Ethylacetat, Acetessigester, Isobutylacetat; Amide, z.B. Formamid, Methylformamid, Dimethylformamid; Ketone, z.B. Aceton, Methylethylketon; und entsprechende Gemische in Betracht. Zweckmäßigerweise

verwendet man das Lösungsmittel in einer Menge von 100 bis 2000 Gew.%, vorzugsweise von 200 bis 700 Gew.%, bezogen auf Ausgangsstoff II bzw. IV.

Beide Verfahren können kontinuierlich oder diskontinuierlich, drucklos oder unter Druck, durchgeführt werden; der Einfachheit halber wird Atmosphärendruck bevorzugt.

Beispiel 1

15,1 Gew.-Teile 3-Keto-1,5-dihydro-thiophen-4-carbonsäuremethylester, 14,2 Gew.-Teile Cyclohexylharnstoff und 0,5 Gew.-Teile p-Toluolsulfonsäure werden in 100 Gew.-Teilen Xylol 4 Stunden unter Rückfluß bei Verwendung eines Wasserabscheiders gekocht. Nach dem Abkühlen wird der Rückstand abgesaugt und aus Toluol umkristallisiert. Man erhält 20,3 Gew.-Teile N-Cyclohexyl-N'-(3-methoxycarbonyl-2,5-dihydro-thien-4-yl)-harnstoff vom Fp. 154 bis 155°C.

Beispiel 2

9,3 Gew.-Teile 3-Amino-4-methoxycarbonyl-1,5-dihydrothiophen-hydrochlorid, 6,0 Gew.-Teile Triethylamin, 7,4 Gew.-Teile Cyclohexylisocyanat und 30 Gew.-Teile Acetonitril werden zusammengegeben und 3 Stunden bei 25°C gerührt. Nach dem Einengen wird der Rückstand mit Wasser gewaschen und aus Toluol umkristallisiert. Man erhält 4,5 Gew.-Teile N-Cyclohexyl-N'-(3-methoxycarbonyl-2,5-dihydro-thien-4-yl)-harnstoff vom Fp. 153 bis 154°C.

Beispiel 3

14,4 Gew.-Teile 3-Keto-3,4-dihydro-thiophen-4-carbonsäuremethylester, 16,1 Gew.-Teile Phenylharnstoff und 0,5 Gew.-Teile Toluolsulfonsäure werden in 90 Gew.-Teilen

Cyclohexan 3 Stunden unter Rückfluß mit einem Wasserabscheider gerührt. Nach dem Abkühlen wird der Rückstand abgesaugt und in Ethanol umkristallisiert. Man erhält 16,1 Gew.-Teile N-Phenyl-N'-(3-methoxycarbonyl-2,5-dihydro-thien-4-yl)-harnstoff vom Fp. 182 bis 184°C.

Entsprechend können beispielsweise folgende Dihydrothiophen-carbonester der Formel I erhalten werden.

| Nr. | $R^1$ | $R^2$ | Fp [°C] |
|---|---|---|---|
| 1 | $CH_3$ | $CH_3$ | 203-212 |
| 2 | $CH_3$ | $C_2H_5$ | 118-120 |
| 3 | $CH_3$ | n-$C_3H_7$ | 160-161 |
| 4 | $CH_3$ | i-$C_3H_7$ | 123-125 |
| 5 | $CH_3$ | n-$C_4H_9$ | 135-137 |
| 6 | $CH_3$ | s-$C_4H_9$ | 174-176 |
| 7 | $CH_3$ | t-$C_4H_9$ | |
| 8 | $CH_3$ | n-$C_5H_{11}$ | |
| 9 | $CH_3$ | i-$C_5H_{11}$ | |
| 10 | $CH_3$ | Cyclohexyl | 154-155 |
| 11 | $CH_3$ | $CH_2CH=CH_2$ | |
| 12 | $CH_3$ | $CH_2C\equiv CH$ | |
| 13 | $CH_3$ | $C_6H_5$ | 168-171 |
| 14 | $CH_3$ | 4-Chlorphenyl | 184-187 |
| 15 | $CH_3$ | 3-Chlorphenyl | 183-185 |
| 16 | $CH_3$ | $CH_3OCH_2CH_2$ | |
| 17 | $CH_3$ | $CH_3SCH_2CH_2$ | |
| 18 | $CH_3$ | $ClCH_2CH_2$ | 133-137 |
| 19 | $CH_3$ | $CH_3CH(Cl)CH_2$ | 136-139 |
| 20 | $CH_3$ | $(CH_3)_2NCH_2CH_2$ | |
| 21 | H | $CH_3$ | |
| 22 | H | $C_2H_5$ | |
| 23 | H | n-$C_3H_7$ | |

| Nr. | $R^1$ | $R^2$ | Fp [°C] |
|---|---|---|---|
| 24 | H | $i-C_3H_7$ | |
| 25 | H | $n-C_4H_9$ | |
| 26 | H | $i-C_4H_9$ | |
| 27 | $C_2H_5$ | $CH_3$ | 154-157 |
| 28 | $C_2H_5$ | $C_2H_5$ | |
| 29 | $C_2H_5$ | $n-C_3H_7$ | |
| 30 | $C_2H_5$ | $i-C_3H_7$ | |
| 31 | $C_2H_5$ | $n-C_4H_9$ | |
| 32 | $n-C_3H_7$ | $CH_3$ | |
| 33 | $n-C_3H_7$ | $C_2H_5$ | |
| 34 | $n-C_3H_7$ | $C_6H_{11}$ | |
| 35 | $i-C_3H_7$ | $CH_3$ | 154-157 |
| 36 | $i-C_3H_7$ | $C_2H_5$ | |
| 37 | $i-C_3H_7$ | $n-C_3H_7$ | |
| 38 | $i-C_3H_7$ | $i-C_3H_7$ | |
| 39 | $n-C_4H_9$ | $CH_3$ | |
| 40 | $n-C_4H_9$ | $n-C_3H_7$ | |
| 41 | $n-C_4H_9$ | $C_2H_5$ | |
| 42 | Cyclohexyl | $CH_3$ | |
| 43 | Cyclohexyl | $C_2H_5$ | |
| 44 | Phenyl | $CH_3$ | |
| 45 | Phenyl | $C_2H_5$ | |
| 46 | Phenyl | $i-C_3H_7$ | |
| 47 | Phenyl | $n-C_3H_7$ | |
| 48 | 4-Chlorphenyl | $CH_3$ | |
| 49 | 3-Chlorphenyl | $CH_3$ | |
| 50 | 4-Fluorphenyl | $CH_3$ | |
| 51 | 4-Isopropylphenyl | $CH_3$ | |
| 52 | $CH_3$ | $s-C_4H_9$ | 143-144 |
| 53 | $CH_3$ | $-CH(C_2H_5)-C_2H_5$ | 190-192 |
| 54 | $CH_3$ | $-CH(CH_3)-CH_2-CH(CH_3)_2$ | 126-129 |

Die Dihydrothiophen-carbonester der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emul-

giermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und

Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono--ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 5 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 1 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 3 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht

wurde, innig vermischt. Man erhält auf diese Weise eine Aufbereitung des Wirkstoffs mit guter Haftfähigkeit.

VIII. 20 Teile der Verbindung Nr. 3 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Vorzugsweise werden die neuen Wirkstoffe nach dem Auflaufen ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bodenart, Jahreszeit, Zielpflanzen und Wachstumsstadium 0,05 bis 5 kg/ha und mehr, vorzugsweise 0,25 bis 3,0 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt

flach eingesät. Unmittelbar danach werden die Wirkstoffe bei Vorauflaufbehandlung auf die Erdoberfläche aufgebracht. Sie werden hierzu in Wasser als Verteilungsmittel suspendiert oder emulgiert und mittels fein verteilender Düsen gespritzt. Die Aufwandmengen betragen 3,0 kg Wirkstoff/ha. Nach dem Aufbringen der Mittel werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt ein gleichmäßiges Keimen der Testpflanzen, sofern dies nicht durch die Wirkstoffe beeinträchtigt wird.

Für die Nachauflaufbehandlung werden die Testpflanzen je nach Wuchsform erst bis zu einer Wuchshöhe von 3 bis 15 cm angezogen und danach behandelt. Die für die Nachauflaufanwendung eingesetzten Soja- und Reispflanzen werden in einem mit Torfmull (peat) angereicherten Substrat angezogen, um ein günstigeres Wachstum zu gewährleisten. Zur Nachauflaufbehandlung werden entweder direkt gesäte und in den gleichen Gefäßen aufgewachsene Pflanzen ausgewählt, oder aber sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,5 bis 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 30°C) und für solche gemäßigter Klimate 15 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0

bis 100. Dabei bedeutet 0 keine Schädigung oder normaler Auflauf und 100 kein Aufgang der Pflanzen bzw. völlige Zerstörung zumindest der oberirdischen Teile.

Die Testpflanzen setzen sich aus folgenden Arten zusammen: Aràchys hypogaea (Erdnüsse), Cassia tora, Chenopodium album (Weißer Gänsefuß), Echinochloa crus-galli (Hühnerhirse), Glycine max. (Soja), Gossypium hirsutum (Baumwolle), Lamium spp. (Taubnessel), Malva neglecta (Wegmalve), Oryza sativa (Reis), Sida spinosa, Sinapis alba (Weißer Senf), Solanum nigrum (Schwarzer Nachtschatten).

Bei Vorauflaufanwendung zeigt beispielsweise die Verbindung Nr. 4 bei einer Aufwandmenge von 3,0 kg Wirkstoff/ha eine gute herbizide Aktivität, insbesondere gegen Senf. Bei Nachauflaufanwendung bekämpfen beispielsweise die Verbindungen Nr. 1, 3 und 5 mit 1,0 kg Wirkstoff/ha unerwünschte Pflanzen und sind gleichzeitig selektiv für Kulturpflanzen. Verbindung Nr. 2 bekämpft mit 0,5 kg/Wirkstoff/ha breitblättrige Unkräuter in Erdnüssen.

In Anbetracht der Verträglichkeit und der Vielseitigkeit der Applikationsmethoden, können die erfindungsgemäßen Verbindungen noch in einer weiteren Zahl von Kulturpflanzen zur Beseitigung unerwünschter Pflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |

| Botanischer Name | Deutscher Name |
|---|---|
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |

| Botanischer Name | Deutscher Name |
| --- | --- |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Zea mays | Mais |

Zur Verbreiterung des Wirkungsspektrums und zur Erzielung synergistischer Effekte können die Dihydrothiophen-carbonester der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Dihydrothiophen-carbonester der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

## Patentansprüche

1. Dihydrothiophen-carbonester der Formel

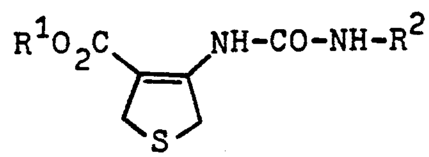

(I),

in der
$R^1$ Wasserstoff, $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl, oder gegebenenfalls durch Halogen substituiertes Benzyl und
$R^2$ $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl, oder gegebenenfalls durch Halogen substituiertes Benzyl bedeuten.

2. Dihydrothiophen-carbonester der Formel I gemäß Anspruch 1, dadurch gekennzeichnet, daß $R^1$ und $R^2$ $C_1$-$C_4$-Alkyl bedeuten.

3. Verfahren zur Herstellung von Dihydrothiophen-carbonestern der Formel I gemäß Anspruch 1, dadurch gekennzeichnet, daß man

a) einen Ketoester der Formel

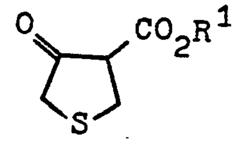

(II),

in der
$R^1$ die in Anspruch 1 genannten Bedeutungen hat,

mit einem Harnstoff der Formel

$$R^2-NH-\overset{O}{\overset{\|}{C}}-NH_2$$ (III),

in der
$R^2$ die im Anspruch 1 genannte Bedeutung hat, gegebenenfalls in Anwesenheit eines Kondensationsmittels umsetzt oder

b) eine Aminoverbindung der Formel

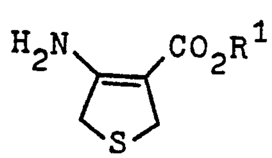

(IV),

in der
$R^1$ die im Anspruch 1 genannten Bedeutungen hat, oder Salze dieser Aminoverbindung

mit einem Isocyanat der Formel

$$R^2\text{-NCO} \qquad (V),$$

in der
$R^2$ die im Anspruch 1 genannten Bedeutungen hat, gegebenenfalls in Gegenwart einer Base in einem inerten Lösungsmittel umsetzt.

4. Herbizid, enthaltend einen Dihydrothiophen-carbonester der Formel I gemäß Anspruch 1.

5. Herbizid, enthaltend inerte Zusatzstoffe und einen Dihydrothiophen-carbonester der Formel I gemäß Anspruch 1.

6. Herbizid gemäß Anspruch 4, dadurch gekennzeichnet, daß $R^1$ und $R^2$ in Formel I des Dihydrothiophen-carbonesters $C_1$-$C_4$-Alkyl sind.

7. Herbizid gemäß Anspruch 4, dadurch gekennzeichnet, daß es 0,1 bis 95 Gew.% an Dihydrothiophen-carbonester der Formel I enthält.

8. Verfahren zur Bekämpfung unerwünschten Pflanzenwuchses, dadurch gekennzeichnet, daß man die unerwünschten Pflanzen und/oder die von unerwünschtem Pflanzenwuchs freizuhaltenden Flächen mit einer herbizid wirksamen Menge eines Dihydrothiophen-carbonsäureesters der Formel I gemäß Anspruch 1 behandelt.

Europäisches Patentamt

**EUROPÄISCHER RECHERCHENBERICHT**

0090309

Nummer der Anmeldung

EP 83 10 2768

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 033 454 (DIAMOND SHAMROCK CORP.) * Seite 4; Seite 16, Beispiel 2; Seite 34, Absatz 2; Seite 36, 2. Testverbindung; Seite 60 * | 1,4 | C 07 D 333/38<br>A 01 N 47/36 |
| A | DE-A-2 122 636 (ESSO) * Ansprüche * | 1,4,8 | |
| A | US-A-2 453 564 (B.R. BAKER) * Ansprüche * | 1 | |
| A | US-A-3 931 204 (P. CROISIER) * Spalte 8, Zeilen 50-60 * | 1,3 | |

RECHERCHIERTE SACHGEBIETE (Int. Cl. 3)

C 07 D 333/00
A 01 N 47/00
A 01 N 43/00

Der vorliegende Recherchenbericht wurde für alle Patentansprüche erstellt.

| Recherchenort | Abschlußdatum der Recherche | Prüfer |
|---|---|---|
| DEN HAAG | 16-06-1983 | CHOULY J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82